# EUROPEAN PATENT APPLICATION

(11) **EP 3 639 820 A1**
(43) Date of publication of application: **22.04.2020**
(21) Application number: 18200610.6
(22) Date of filing: 16.10.2018
(51) Int. Cl.: A61K 31/343, A61P 35/00

(54) **FLAVAGLINE DERIVATIVES FOR INHIBITION OF KRAS ONCOGENE ACTIVATION**

(71) Applicant: Universitätsmedizin der Johannes Gutenberg-Universität Mainz, 55131 Mainz (DE)
(72) Inventor: RAJALINGAM, Krishnaraj, 55268 Nieder-Olm (DE); YURUGI, Hajime, 55118 Mainz (DE)
(74) Representative: Patentanwälte Dr. Keller, Schwertfeger Partnerschaft mbB

(57) **Abstract**

The present invention relates to inhibitors of KRAS oncogene activation, which are flavagline derivatives with the ability to target prohibitin to inhibit KRAS activation.

The flavagline derivatives according to the invention have the general formula (I) 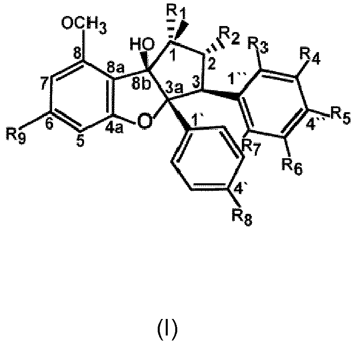 in which
R1 is HO, O-O, HNO, HNON(CH₃)₂, HNONH₂;
R2 is H, COOCH₃, CONHCH₃, NO(CH₃)₂;
R3, R4, R5 are each independently H, OH;
R6 is H, OH, F;
R7 is H, OH;
R8 is H, OCH₃, Br, F, Cl;
R9 is OCH₃, CH₃HN.

The invention further relates to pharmaceutical compositions comprising one or more of said flavagline derivatives and the use in methods for inhibition of KRAS activation.

## Description

The present invention relates to novel inhibitors of KRAS oncogene activation, which are flavagline derivatives with the ability to target prohibitin to specifically and effectively inhibit KRAS oncogene activation. The invention further relates to pharmaceutical compositions comprising one or more of said flavagline derivatives and their use in methods for inhibition of KRAS activation both in vitro and ex vivo. The invention further relates to use of said flavagline derivatives in the prevention or treatment of a disease in which RAS signalling is pathologically involved, in particular a KRAS-mutated proliferative disorder or genetic disorder.

RAS proteins represent a group of closely related monomeric globular proteins which are associated with the plasma membrane and are able to bind either GDP or GTP. RAS that contains bound GDP represents the "inactive" state, whereas the binding of GTP to RAS in exchange to a GDP represents the "active" state, such that the protein is able to interact with other proteins of downstream targets. RAS proteins can be regarded as small GTPases that function as molecular switches controlling the transmission of extracellular signals from outside of the cell to the nucleus by various effector proteins (Downward, J. Targeting RAS signalling pathways in cancer therapy. Nat Rev.Cancer 3, 11-22 (2003)).There are three RAS isoforms (KRAS, HRAS and NRAS) and their activation cycle is regulated by the binding of GDP or GTP which in turn is controlled by GAPs or GEFs (Rajalingam, K., Schreck, R., Rapp, U. R. & Albert, S. Ras oncogenes and their downstream targets. Biochim.Biophys.Acta 1773, 1177-1195 (2007)). In their GTP-bound form they bind to their effector proteins and trigger multiple signalling pathways that control various fundamental cellular processes. Almost 30% of human cancers carry gain of function mutations in the RAS oncogene and among the three isoforms, KRAS (85%) is one of the most frequently mutated oncogenes followed by NRAS (11%) and HRAS (4%) (COSMIC database). Numerous studies have demonstrated a role for RAS as an "oncogenic driver" in several cancers (Hobbs, G. A., Der, C. J. & Rossman, K. L. RAS isoforms and mutations in cancer at a glance. J Cell Sci 129, 1287-1292, doi:10.1242/jcs.182873 (2016)). Usually, mutations of RAS lead to defects in GAP-mediated GTP hydrolysis and thus resulting in the accumulation of RAS in the GTP-bound active state. Most of the mutations in RAS isoforms affect three hotspot residues (G12, G13 and Q61) and recent studies revealed that the biological consequences of these mutations are not identical (Miller, M. S. & Miller, L. D. RAS Mutations and Oncogenesis: Not all RAS Mutations are Created Equally. Front Genet 2, 100, doi:10.3389/fgene.2011.00100 (2011)).

Despite intense efforts for three decades, drugs directly targeting RAS oncogenes have not reached clinical application. The three RAS isoforms exhibit 82-90% sequence identity and most of the differences are confined to the C-terminal HVR region. Whether amino acid sequence divergence in the HVR region directly contributes to the differences in the biological responses elicited by these three RAS isoforms is not well understood. Through alternative splicing, two KRAS isoforms are generated (KRAS4A and KRAS4B) with KRAS4B being predominantly expressed in cancers. While KRAS mutations are frequently identified in pancreatic, lung and colon carcinomas, NRAS mutations often occur in melanomas and HRAS mutations predominate in head and neck cancers. The underlying mechanisms behind the RAS isoform specific mutations and their tissue specific roles are currently unclear. Further, there are significant differences between the RAS isoforms with respect to their posttranslational modifications and intracellular localization (Simanshu, D. K., Nissley, D. V. & McCormick, F. RAS Proteins and Their Regulators in Human Disease. Cell 170, 17-33, doi:10.1016/j.cell.2017.06.009 (2017)).

KRAS4A and KRAS4B have polybasic stretches that are responsible for their affinity to phospholipids in lipid nanoclusters of the plasma membrane (Zhou, Y. & Hancock, J. F. Ras nanoclusters: Versatile lipid-based signalling platforms. Biochim Biophys Acta 1853, 841-849, doi:10.1016/j.bbamcr.2014.09.008 (2015)). KRAS4B can also be phosphorylated at S181 which might dictate its localisation to endomembranes (Prior, I. A. & Hancock, J. F. Ras trafficking, localization and compartmentalized signalling. Semin Cell Dev Biol 23, 145-153, doi:10.1016/j.semcdb.2011.09.002 (2012)). While HRAS is palmitoylated at two residues, KRAS4A and NRAS are palmitoylated at a single residue in the HVR region. These modifications in turn determine their distribution into the plasma membrane microdomains which in turn dictate the downstream signalling.

Because KRAS is the most commonly mutated oncogene in human cancer, with particularly high frequency in cancers of the pancreas, colon, and lung, efforts have been taken to evolve therapeutic strategies. WO 2017/058768 A1 describes compounds having activity as inhibitors of G12C mutant KRAS protein. Similar compounds are also described in US 2014/288045 A1. KRAS mutation is associated with pure prognosis, yet there are no effective therapies to specifically treat cancers expressing mutant forms of the KRAS oncoprotein.

Recent studies revealed that flavaglines, which are natural anti-tumour drugs, directly target prohibitin 1 (PHB1)-CRAF interaction, resulting in CRAF inactivation leading to a block in MAPK pathway which is required for RAS-mediated tumourigenesis. As the structure of the kinase domains is similar, small molecule kinase inhibitors often lead to undesired side effects.

Furthermore, cancer patients frequently develop resistances to kinase inhibitors (Lovly CM, Shaw AT. Molecular pathways: resistance to kinase inhibitors and implications for therapeutic strategies. Clin Cancer Res 2014; 20: 2249-2256). Thus, targeting oncogenic kinase outside the kinase domain or protein-protein interaction domain to inactivate an oncogenic kinase will be an attractive strategy to combat human cancers or other KRAS signalling-related disorders.

Although a treatment with rocaglamide inhibits RAS-activation in KRAS-mutated cell lines, the substance is not specific enough to differentiate between KRAS and a closely related isoform HRAS and NRAS (H Yurugi et al., targeting prohibitants with chemical ligands inhibits KRAS-mediated lung tumours, oncogene (2017), Vol. 36:4778-4789)).

It is therefore the object of the present invention to provide compounds that have the capability to inhibit the activation of KRAS in cells at nanomolar concentrations with high specificity. This object is solved by the flavagline derivatives as claimed in claim 1. Preferred embodiments of the invention are part of the dependent claims.

The novel flavagline derivatives of the present invention qualify as inhibitors of RAS oncogene activation by inhibiting the prohibitin pathway, in particular inhibiting EGF-induced RAS-GTP loading in cells. The inventors further show that the flavagline derivatives of the present invention compromise GTP loading on KRAS in cells carrying mutated KRAS at nanomolar concentrations. In order to identify potential candidate substances, the inventors conducted a screening of flavagline compounds that are able to inhibit the activation of RAS by directly disrupting the interaction between activated KRAS (both by EGF and mutational activation) and the Ras binding domain (RBD) of the CRAF kinase in cells. As one outcome of this screening, the inventors identified several derivatives of flavaglines that qualify as inhibitors of KRAS. The identified flavagline compounds are based on a common core structure but differ from each other by different side chains.

The term "flavagline derivatives" as used in the present invention relates to a group of flavaglines that inhibit GTP-loading of KRAS, thereby leading to inactivation of GTPase.

The flavagline derivatives of the present invention comprise a cyclopentabenzofuran ring which can also be found in natural flavaglines isolated from plants of the genus Aglaia (Basmadjian, C., Thuaud, F., Ribeiro, N. & Desaubry, L. Flavaglines: potent anticancer drugs that target prohibitins and the helicase eIF4A. Future Med Chem 5, 2185-2197, doi:10.4155/fmc.13.177 (2013)). Accordingly, the flavagline derivatives that have the desired activity according to the present invention are based on the following general formula (I) 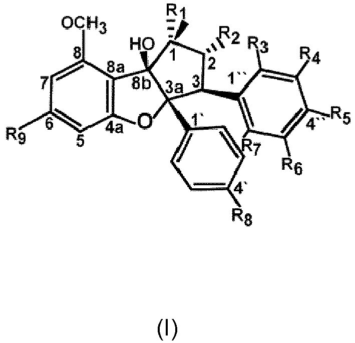 in which
R1 is HO, O-O, HNO, HNON(CH₃)₂, HNONH₂;
R2 is H, COOCH₃, CONHCH₃, NO(CH₃)₂;
R3, R4, R5 are each independently H, OH;
R6 is H, OH, F;
R7 is H, OH;
R8 is H, OCH₃, Br, F, Cl;
R9 is OCH₃, CH₃HN.

Most surprisingly, only a specific set of flavaglines is able to specifically inhibit KRAS, at very low nanomolar concentrations. This qualifies the inventive compounds as being effective therapeutic agents in the treatment of diseases in which RAS signalling is pathologically involved. The inventive flavagline derivatives efficiently inhibit KRAS activation and prevent the oncogenic growth of tumour cells both in vitro and in vivo, as shown by using animal models.

The most potent KRAS inhibitors according to the present invention besides rocaglamide are FL3, FL10, FL13, FL15, FL19, FL23, FL32, FL37, FL40, FL42, IMD-26260 having the following structures

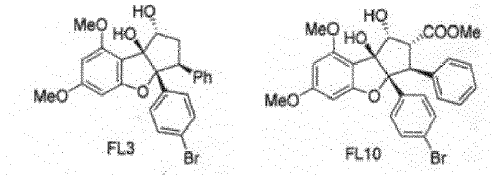

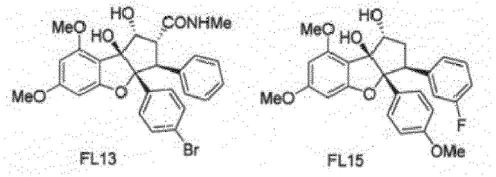

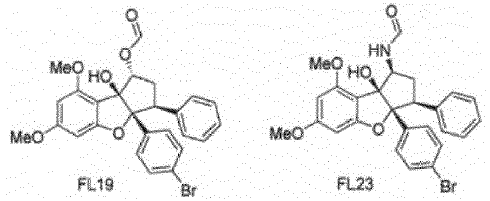

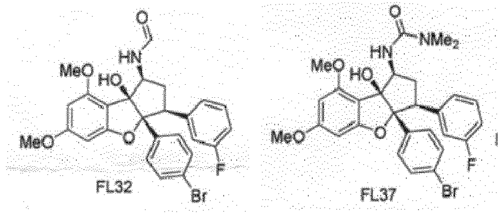

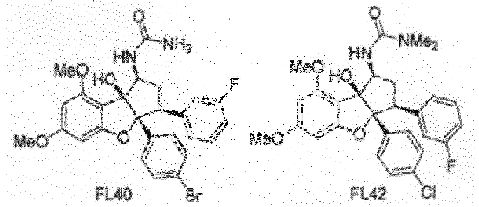

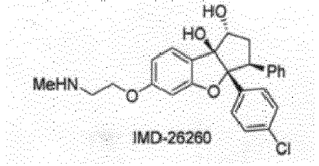

The flavagline derivatives of the present invention efficiently inhibit nano-clustering of KRAS, thereby preventing effector protein activation. These effects were re-produced by the depletion of prohibitin, suggesting a direct role of the prohibitin in the regulation of KRAS activation. Even more specifically, the inventive compounds inhibit the interaction of specific KRAS mutants with CRAF-RBD, in particular mutated KRAS genes having a KRAS G12V, G12C, G12D, G13C, G13D, G13S, Q61H, Q61R, Q61K mutations. Surprisingly, nanomolecular concentrations are sufficient for the flavagline compounds of the invention to elicit the desired inhibitory effect on mutated KRAS. As revealed by a nano-bit assay and conducting kinetics experiments, KRAS activation is effectively inhibited at an IC50 of 25 nM.

In another aspect, the present invention relates to the use of the identified flavagline derivatives for inhibiting KRAS activation in cells in vitro or ex vivo. In a preferred embodiment, the compounds of the present invention can be used in the method of specifically inhibiting proliferation of a cell population in vitro or ex vivo by specifically targeting KRAS. In preferred variants of the invention KRAS4A or KRAS4B is specifically inhibited, but not HRAS or NRAS nanoclustering. The method comprises the step of contacting the cell population with a flavagline derivative of the present invention. Preferably, inhibition of the proliferation of a cell population is measured as a decrease in cell viability of the cell population.

The present invention also relates to a pharmaceutical composition, comprising a compound which is a flavagline derivative having the general formula (I) 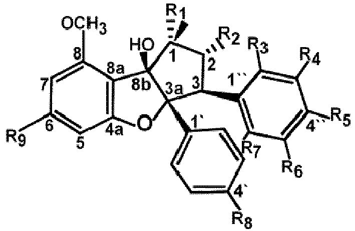 in which
R1 is HO, O-O, HNO, HNON(CH₃)₂, HNONH₂;
R2 is H, COOCH₃, CONHCH₃,NO(CH₃)₂;
R3, R4, R5 are each independently H, OH;
R6 is H, OH, F;
R7 is H, OH;
R8 is H, OCH₃, Br, F, Cl;
R9 is OCH₃, CH₃HN;
and a pharmaceutically acceptable vehicle, diluent, adjuvant or excipient.

In a preferred embodiment, the pharmaceutical composition comprises a flavagline derivative, which is FL3, FL10, FL13, FL15, FL19, FL23, FL32, FL37, FL40, FL42, IMD-26260 having the following chemical structures:
In a preferred embodiment, the pharmaceutically acceptable vehicle, diluent, adjuvant 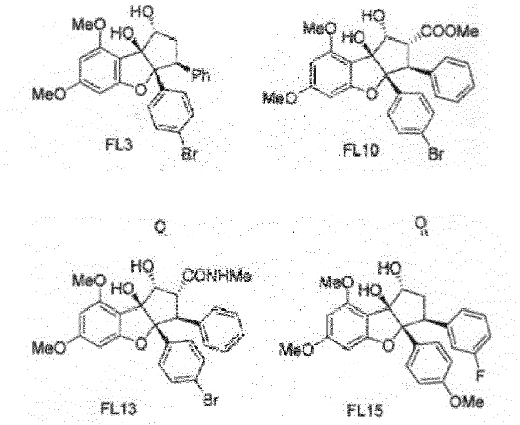 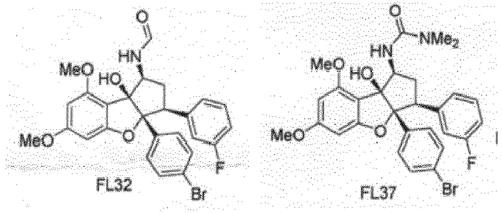 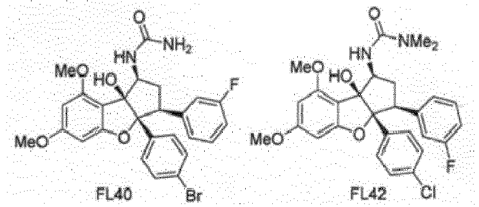 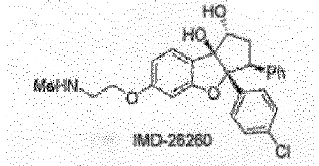 or excipient can be any carrier, diluent, preservative, dye/colorant, flavour enhancer, surfactant, dispersing agent, suspending agent, stabilizer, isotonic agent, solvent, or emulsifier which is known for the person skilled in the art and being acceptable for use in humans or domestic animals.

A "pharmaceutical composition" according to the invention refers to a formulation of one or more compounds of the invention and a medium generally accepted in the art for the delivery of the biologically active compound to mammals, e.g. humans or animals.

The term "ex vivo" in the context of the present invention refers to an event that plays outside of the human or animal body.

As shown by the present invention, treatment with the flavagline derivatives of the present invention directly inhibit KRAS GTP loading in cells, which makes the compounds suitable as biologically and pharmaceutically effective agents for use in the prevention or treatment of a disease in which RAS signalling is pathologically involved, in particular a KRAS-mutated proliferative disorder or genetic disorder. Such a disease can be any of a neoproliferative disease, cancer, RASopathies or craniofacial syndrome. In particular, any cancer in which mutated KRAS or other RAs isoforms plays a role is a suitable target for the compounds of the present invention, preferably a cancer such as colorectal cancer, lung cancer, hematological cancer, MYH associated polyposis, bladder cancer, melanoma, acute myeloid leukemia (AML), or pancreatic cancer. Preferred targets of a disease in which RAS signalling is pathologically involved, are mutated KRAS genes, in particular such ones that carry a G12V, G12C, G12D, G13C, G13D, G13S, Q61H, Q61R, Q61K mutation. However, also other KRAS mutants that are sensible for KRAS inhibition, in particular KRAS mutants in which the interaction to CRAF-RBD or other Ras effector proteins is inhibited by a flavagline derivative, are encompassed by the present invention.

The present invention is illustrated in more detail in the following examples.

### EXAMPLES

The following experiments show the in vitro and in vivo effects of the flavagline derivatives of the present invention.

In the following, the inventors show that the targeting plasma membrane-associated prohibitins with the flavagline derivatives of the present invention inhibit the GTP-loading of KRAS, resulting in an inactivation of GTPase activity.

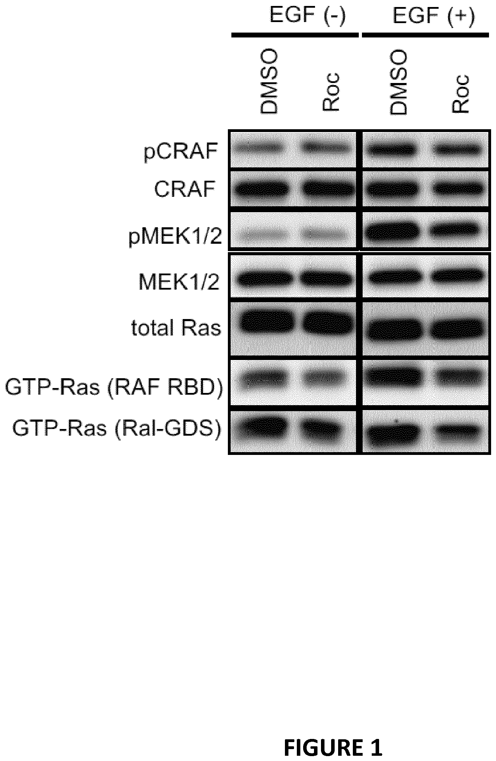

Fig. 1 shows a control experiment to test whether rocaglamide prevents RAS activation in response to EGF in HeLa cells. HeLa cells were treated with Roc for 1h before the EGF stimulation. The left panel shows the blot from EGF-unstimulated condition and the right panel shows the EGF-stimulated condition. GTP-loaded Ras was detected by pull down assay with RAF RBD domain and Rap GDS RA domain (last 2 panels from the bottom). Crypt pull down assays employing CRAF-RBD and the Ral-GDS-RA domains were performed. Activation of RAS in response to EGF was inhibited by pre-treating cells with rocaclamide at 200 nM concentration. Then, a nano-bit assay employing KRAS and CRAF-RBD to quantify the activation of KRAS was established.

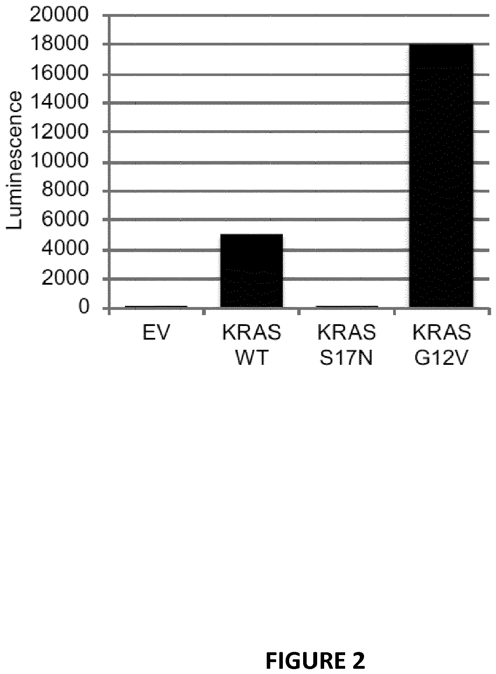

Fig. 2 shows that KRAS S17N, an inactive mutant of KRAS, failed to interact with CRAF-RBD, confirming the sensitivity and robustness of the assay employed. Nano Bit assay for the KRAS GTP-loading was performed in Hela cells. KRAS mutants expression plasmids were transfected to Hela cells and the cell were used for NanoBit assay. For each KRAS mutant, the values from DMSO-treated cells were taken as 1 and the fold inhibition of the compounds is shown in the figure. The bars represent mean ± SEM, from 3 independent experiments.

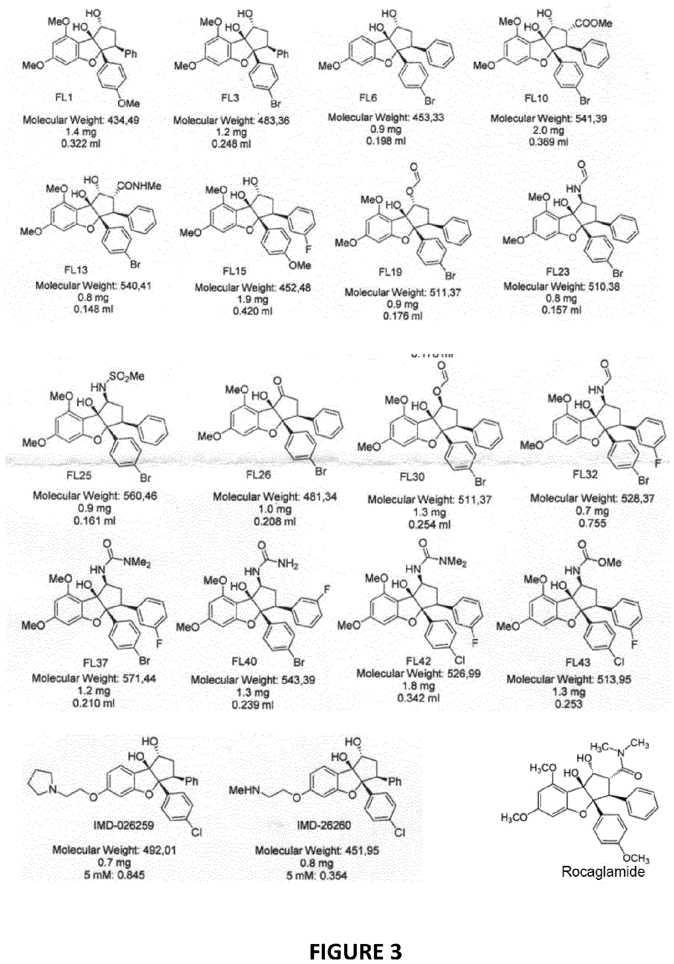

Fig. 3 shows different flavagline derivatives including rocaclamide that were tested in KRAS activity assays to see which of those compounds are able to inhibit (mutated) KRAS activation. The compounds differ by each other in their side chains. Potential candidates were identified by nano-bit assays in which inhibition of KRAS was determined.

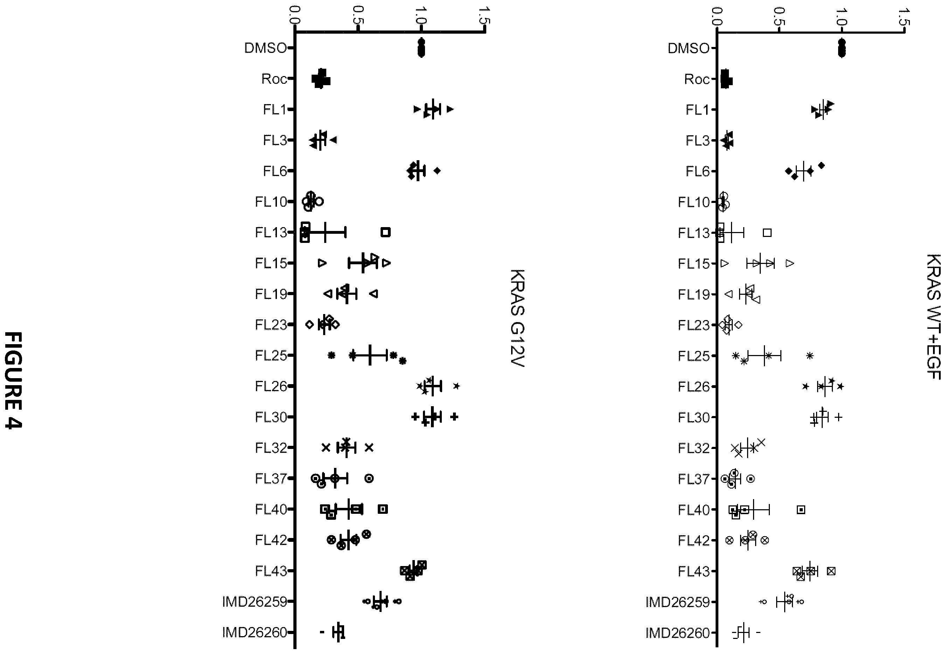

Fig. 4 shows that not all tested compounds result in an inactivation of KRAS, but a subset of flavaglines, in particular FL3, FL10, FL13, FL15, FL19, FL23, FL32, FL37, FL40, FL42, IMD-26260, and also rocaglamide. Few candidates like FL1, FL6, FL26, FL30 failed to inhibit KRAS activation despite the presence of the signature cyclopentabenzofuran ring, suggesting that structural moieties in their side chains contribute to KRAS inhibiting ability. Similar results were obtained when wild type KRAS was activated with EGF. NanoBit system was used to evaluate the effect of compounds. For KRAS WT transfected cells, the cell were treated with 100 nM compounds for 4h in serum free medium followed by EGF stimulation (100ng/ml) for 30min. For KRAS G12V transfected cells, the cells were treated with 100 nM compounds for 4h. After treatment or stimulation, NanoBit assay was performed according to the instruction. Data is showing the results of relative value of luminescence and the value from DMSO treated cells was taken as 1. Bars represent the mean value ± SEM (n=4).

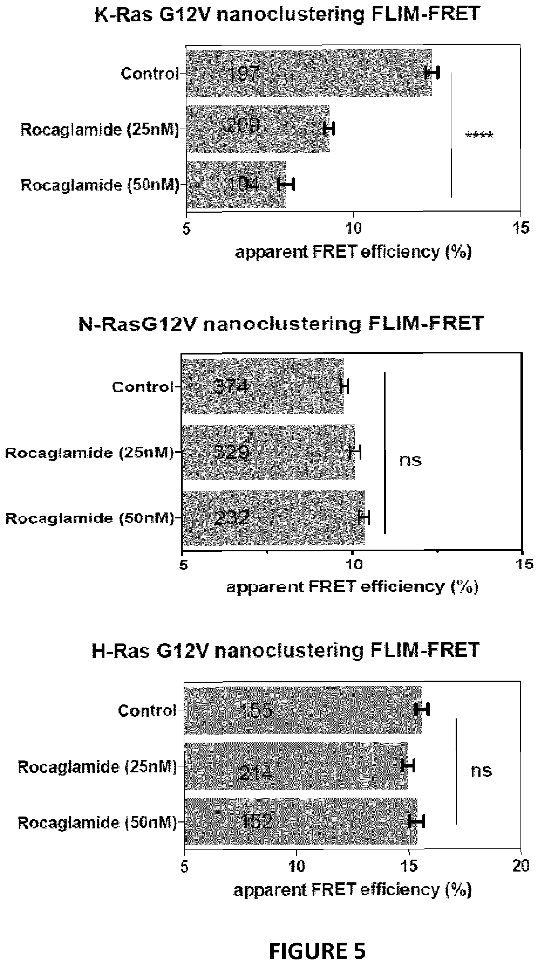

Fig. 5 shows an FRET-FLIM analysis RAS nanoclusters (KRAS, HRAS and NRAS) at the plasma membrane. The flavagline derivatives of the present invention specifically inhibit KRAS nanocluster formation at the plasma membrane, as shown by K-RasG12V, H-RasG12V and N-RasG12V nano-clustering-FRET. HEK-293 EBNA cells were transfected with pmGFP tagged K-RasG12V or H-RasG12V or N-RasG12V (1µg) for donor fluorophore lifetimes. For cells expressing the FRET pairs, cells were co-transfected with pmGFP-KRasG12V (0.5 µg) and pmCherry-KRasG12V (1.5 µg), or pmGFP-H-RasG12V (0.5 µg) and pmCherry-H-RasG12V (1.5 µg), or pmGFP-N-RasG12V (0.5 µg) and pmCherry-N-RasG12V (1.5 µg). After 24 hours of transfection, cells were treated with 0.1% DMSO control or 25 nM or 50 nM of rocaglamide. After 24 hours of treatment cells were fixed with 4% PFA. Numbers on the bars indicate number of analyzed cells. Statistically significant differences between control and treated samples were examined using one-way ANOVA complemented with Tukey's comparison (significant; ****P<0.0001; ns, not significant). The bars represent mean values ± SEM from 3 independent biological experiments.

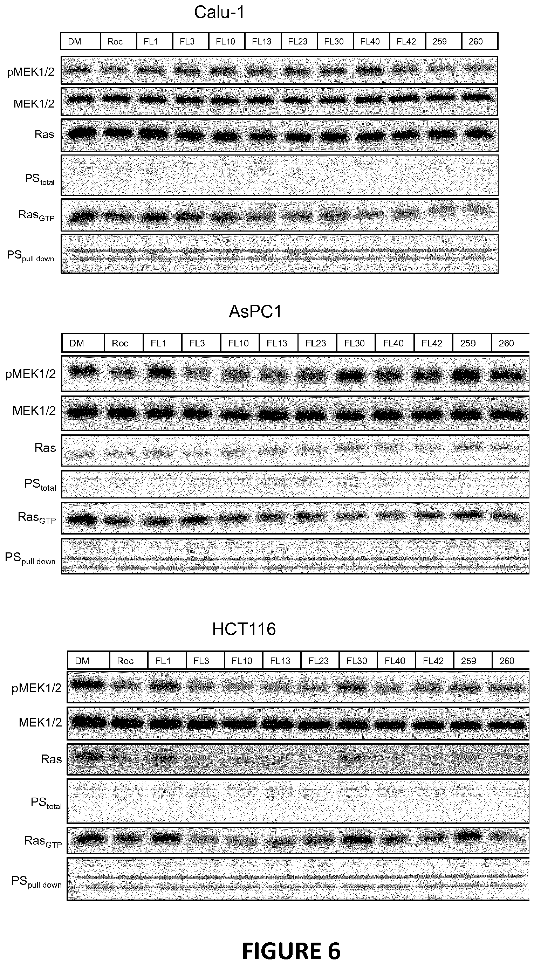

Fig. 6 shows the effect of nanomolar concentrations of the flavagline derivatives (100 nm) on RAS-activation in KRAS-mutated cell lines (Calu-1, ASPC-1 and HCT-116). The activation of RAS was monitored by the crypt pull down assay described above. The activation of CRAF and MEK1 was checked by immunoblot analysis.

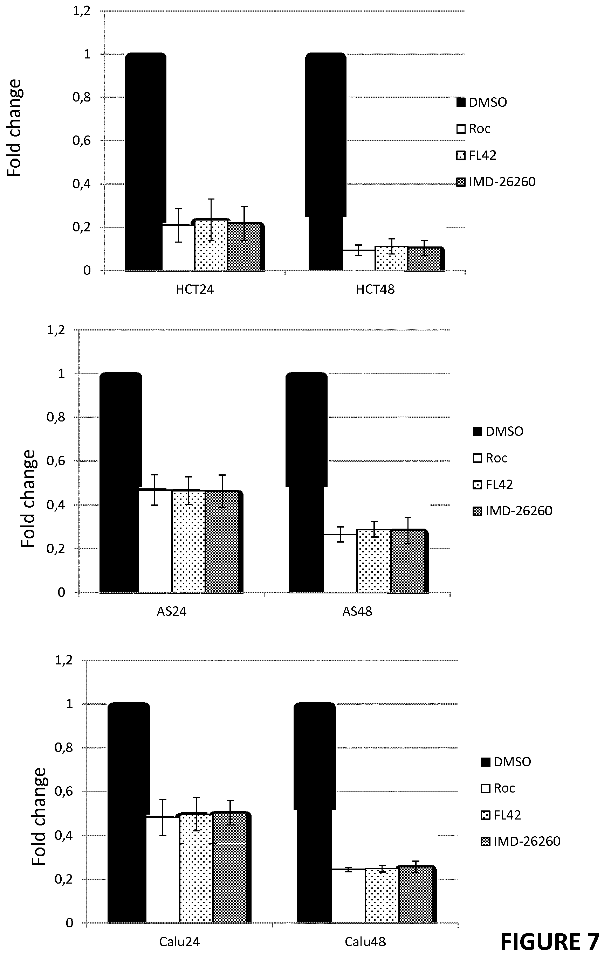

Fig. 7 shows the effects of three flavagline derivatives on the viability of tumour cell lines as tested by a MTT assay. Data are shown from three independent experiments (n=3).

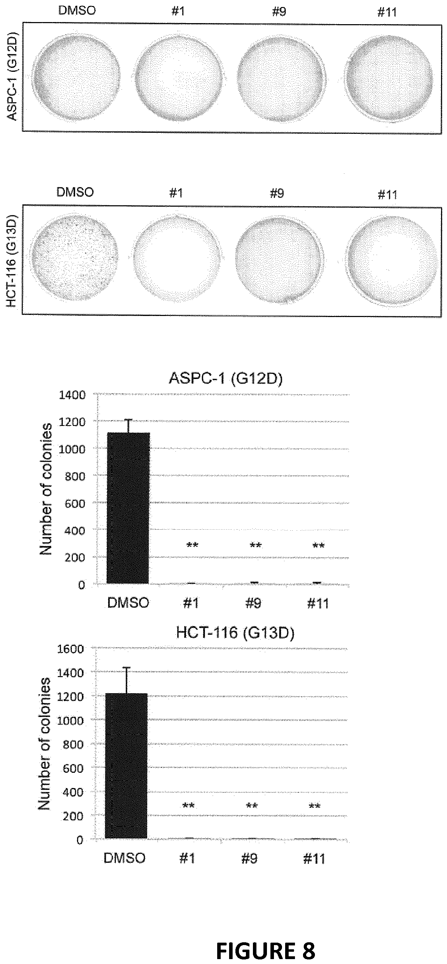

Fig. 8 shows a soft agar colony formation assay revealing the effects of the flavagline derivatives of the present invention. Data from three independent experiments are shown (n=3).

The data exemplify that flavagline derivatives of the present invention disrupt the formation of KRAS nanoclusters, but not HRAS or NRAS nanoclusters. Therefore, the compounds of the present invention are specific for KRAS inhibition, possibly by influencing the prohibitin-dependent segregation of lipids within the plasma membrane.

The data also exemplify that the growth of cancer cells can be inhibited in vitro by employing MTT and soft agar assays. The flavagline derivatives of the present invention are effective in blocking the growth of HCT-116, ASPC-1 and Calu-1 cells, which carry KRAS mutations, with an IC50 in low nanomolecular range (6-20 nM).

These studies were extended in in vivo animal models by employing autochthonous mouse models. A KRAS G12D-driven NSCLC mouse model was used. The expression of KRAS G12D was induced with doxycycline treatment for about 2 months, which was then followed by treatment of the mice with flavagline derivatives of the present invention at 2,5 mg/kg for 6 weeks. Flavagline derivative treatment led to strong reduction in the number of lung nodules, suggesting that the growth and maintenance of KRAS G12D-derived NSCLCs in vivo are successfully inhibited (data not shown).

The data reveal a natural anti-tumor drug that specifically inhibits KRAS at nanomolar concentrations both in in vitro cell culture models as well as in autochthonous mouse models. As such, the compounds of the present invention are suitable for diseases, in which RAS signalling is pathologically involved, in particular a neoproliferative disease, cancer, RASOpathy or craniofacial syndrome. Malignant cancer diseases that are characterized by mutated KRAS are colorectal cancer, lung cancer, hematological cancer, MYH associated polyposis, bladder cancer, melanoma, acute myeloid leukemia (AML), or pancreatic cancer.

### Materials and Methods

### Cells

482T1 cells were a kind gift from Tyler Jack's lab and cultured in DMEM (10 % heat inactivated FBS). Calu-1 cells were obtained from Sigma-Aldrich and cultured in McCoy's 5A medium (10 % heat inactivated FBS). HeLa (DSMZ) and HCT-116 (a gift from Ulf Rapp) were authenticated by Eurofin genomics and cultured in DMEM (10 % heat-inactivated FBS). ASPC-1 cells were purchased from DSMZ and cultured in RPMI-1640 (10 % heat-inactivated FBS). HeLa cells were starved in serum-free medium with rocaglamide or flavaglines for 4 hours and stimulated with EGF (100 ng/ml) for 30min. KRAS mutation carrying cells were treated with Roc in complete growth medium for 24 hours.

### DNA/siRNA Transfection

HeLa cells were harvested with 0.05% Trypsin/0.02% EDTA in PBS and seeded in 6 well or 12 well cell culture plates at the concentration of 5x10⁴ cells in complete DMEM (2 ml for 6 well plate and 1 ml for 12 well plate). After 1 day from seeding, the DNA or siRNAs were mixed with various transfection reagents (described below) and was added to the well for 1-2 days. The medium was changed to serum-free DMEM and incubated at 37°C for 4h with flavaglines (100 nM). After starvation of the cells, the cells were stimulated with EGF (100 ng/ml) for 30min. The cells were then lysed and activated RAS was captured from the cells with RAF-RBD beads as described below. HCT-116 cells were harvested with 0.05% Trypsin/0.02% EDTA in PBS and seeded in 6 well cell culture dishes at the concentration of 5x10⁴ cells/ml, 2ml in 6 well plate. After 1 day, siRNA transfection regent was added to the well and the cells were further cultured for 2 days. Cells were washed with PBS and employed for active Ras pull down assay.

**DNA Transfection regent**

| Plasmid | PEI (10 mM) | PBS |
|---|---|---|
| 1 µg/2 µg (12 well/6 well) | 5 µl/10µl | 100 µl/200 µl |

**siRNA Transfection reagent**

| PHB1: 5'-CCCAGAAAUCACUGUGAAA-3, 5'-UUUCACAGUGAUUUCUGGG. CRAF: 5'-UGUACUACCAUCAACAUCCAC-3' | | |
|---|---|---|
| siRNA (100µM) | Saint Red | HBS |
| 2 µl | 10 µl | 200 µl |

### Active Ras pull down assay

After the stimulation or treatment, active Ras pull down buffer (25 mM Tris-HCl pH7.2, 150 mM NaCl, 5 mM MgCl₂, 1% NP-40, 5% Glycerol with protease inhibitor cocktail) was added to each well and incubated on ice for 30min. Cells were sonicated for 3 seconds, the cell lysates were centrifuged for 15 min at 4°C, 13000 rpm. The protein concentration was adjusted by 660 nm protein assay reagent (Thermoscientific) and 20% of the lysate was collected for the total cell lysate control. 10 µl of CRAF-RBD protein coated agarose beads were added to the rest of lysates and rotated at 4°C for 60min. After incubation, the beads were washed with binding buffer twice and 50 µl of SDS-PAGE sample buffer (125mM Tris-HCI pH 6.8, 4% SDS, 10% Glycerol, BPB) was added.

### SDS-PAGE and western blotting

The samples were loaded onto a 14% SDS-PAGE followed by western blotting. After transfer, the membrane was blocked with 3% BSA/TBST (20mM Tris-HCI, pH7.5, 150mM NaCl, 0.05% Tween-20) for 1h at room temperature. The membrane was incubated with primary antibody diluted in 1% BSA/TBST and incubated over night at 4°C. After the over night incubation, the membrane was washed with TBST (5minx5) and incubated with HRP-conjugated secondary antibody in TBST for 1h at RT. After the secondary antibody treatment, the membrane was washed and the signal was visualized chemiluminescence substrate (Millipore) and Chemidoctouch (Bio-Rad).

### Microscopy analysis

One day after transfection, the cells were harvested and seeded on the cover slips (18mmx18mm) and cultured for 24 h. The medium was changed to serum-free DMEM and incubated at 37°C for 4h with compound (100 nM). After starvation, the cells were stimulated with EGF (100 ng/ml) for 30min. The slide was fixed with Riti HistoFix for 15min at RT, followed by permeabilization with 0.1% TritonX-100 in PBS for 3min and the slide was blocked with 0.5% BSA/PBS for 1h at RT. The cover slip was incubated with primary antibody (1/500, anti-FLAG M2 antibody, Sigma) in 0.5% BSA/PBS for 2h at RT. Subsequently, it was incubated with Cy3 conjugated anti-mouse IgG with Hoechst (5µg/ml) in 0.5% BSA/PBS for 1h at RT. The cover slip was washed with PBS (5 times) and mounted to the glass slide with Mowiol/DABCO solution.

### NanoBit assay

N-terminal LgBit and C-terminal smBit construct was purchased from Promega and the KRAS (full length) was cloned with Xho I and Bgl II to LgBit and CRAF-RBD(1-149) was cloned with EcoRI and Bgl II to SmBit. The constructs were transfected to HeLa cells and the cells were harvested after 1 day of transfection and seeded to 96 well white plate. The cells were cultured in 96 well plate for 1 day and then the medium was changed to serum free DMEM for 0-4h with compounds. After pre-treatment, KRAS WT-transfected cells were stimulated with EGF for 30min. Nano Glo assay was performed for the EGF-stimulated KRAS WT transfected cells or KRAS G12V-transfected cells following manufacturer's instructions. The luminescence was measured using Tecan infinite (Tecan).

### MTT assay

HCT-116, Calu-1 and ASPC-1 cells were seeded in 96 well plates at the concentration of 5x10⁴ cells/ml, 50 µl in 96 well cell culture plate and cultured for 1 day. 50 µl of compound containing growth medium was added to the well and the plate was cultured for 48h. 10 µl of MTT solution was added to the wells and incubated for 2-3 hours. After incubation with MTT, solubilization buffer was added and incubated over night. MTT was measured at O.D. 570 nm with a plate reader (TECAN).

### Soft agar colony formation assay

1.5% agarose solution was mixed with 2X growth medium (20% FBS, with or without 100 nM rocaglamide) and placed with 1.5 ml of 0.75% agarose/1x growth medium in 6 well plate and incubate at room temperature for at least 10 min to solidify agarose. HCT-116 and ASPC-1 cells were diluted in 2X growth medium (20% FBS, with or without 100 nM rocaglamide) and mixed with 0.9% agarose solution. 1.5 ml of cell suspension in 0.45% agarose in 1x growth medium was added to the bottom agarose layer. The cells seeded in soft agar were cultured for 2-4 weeks followed by the staining with crystal violet solution. The images were taken under a ChemiDoc Touch (Bio-Rad) equipment and the number of colonies was counted by image J software.

### Autochthonous mouse models

The autochthonous models was generated and kindly gifted by Dr. Bockamp. SP-C/rtTA (SP-C) mice generated by Dr. Jeffrey A. Whitstett were crossed against Tet-op-K-Ras4b^{G12D} (K-Ras^{G12D}) mice¹⁶ (Fisher, Wellen et al. 2001). For transgene activation mice were fed with DOX-containing food. To prepare the DOX diet, 3g of DOX were dissolved in 3l ddH₂O. 900 ml of DOX solution (600mg/ml) was then added to 2kg food pellets. The soaked pellets were incubated at 37°C for two days and the dry diet was used for feeding. For the development of the tumor, the mice were feed with dox diet for 2 month before the treatment and rocaglamide (2.5mg/kg) was given to mice for 6 weeks intraperitoneally.

## Claims

1. An inhibitor of KRAS oncogene activation, which is a flavagline derivative having the general formula (I) 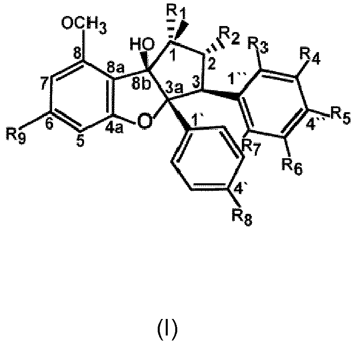 in which
R1 is HO, O-O, HNO, HNON(CH₃)₂, HNONH₂;
R2 is H, COOCH₃, CONHCH₃, NO(CH₃)₂;
R3, R4, R5 are each independently H, OH;
R6 is H, OH, F;
R7 is H, OH;
R8 is H, OCH₃, Br, F, Cl;
R9 is OCH₃, CH₃HN.

2. The inhibitor of KRAS oncogene activation according to claim 1, wherein the flavagline derivative is FL3, FL10, FL13, FL15, FL19, FL23, FL32, FL37, FL40, FL42, IMD-26260 having the following structures:

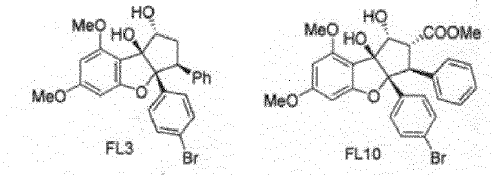

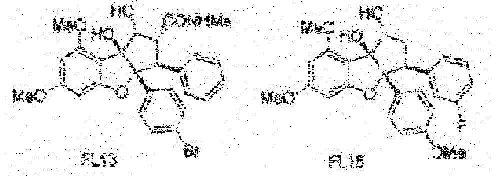

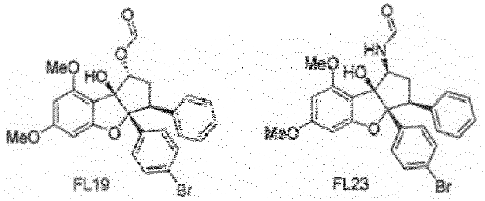

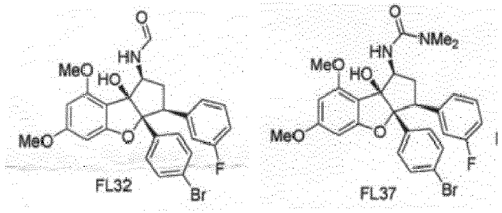

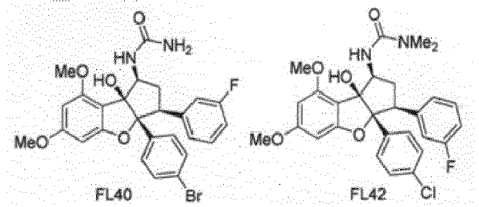

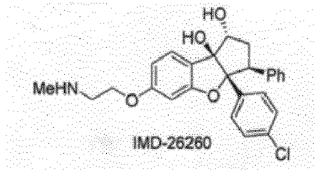

3. Use of a compound comprising anyone of the structures as defined in claim 1 or claim 2 for inhibiting KRAS activation in cells in vitro or ex vivo.

4. A method of specifically inhibiting proliferation of a cell population in vitro or ex vivo by targeting KRAS, the method comprising the step of contacting the cell population with a compound as defined in anyone of claims 1 to 2.

5. The method according to claim 4, wherein inhibition of the proliferation of the cell population is measured as a decrease in cell viability of the cell population.

6. A pharmaceutical composition, comprising a compound, which is a flavagline derivative having the general formula (I) 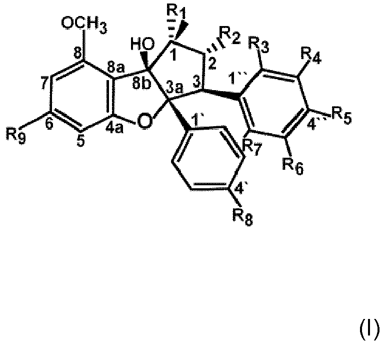 in which
R1 is HO, O-O, HNO, HNON(CH₃)₂, HNONH₂;
R2 is H, COOCH₃, CONHCH₃, NO(CH₃)₂;
R3, R4, R5 are each independently H, OH;
R6 is H, OH, F;
R7 is H, OH;
R8 is H, OCH₃, Br, F, Cl;
R9 is OCH₃, CH₃HN;
and a pharmaceutically acceptable vehicle, diluent, adjuvant or excipient.

7. The pharmaceutical composition according to claim 6, wherein the flavagline derivative is FL3, FL10, FL13, FL15, FL19, FL23, FL32, FL37, FL40, FL42, IMD-26260 having the following structures

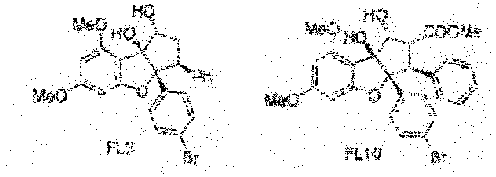

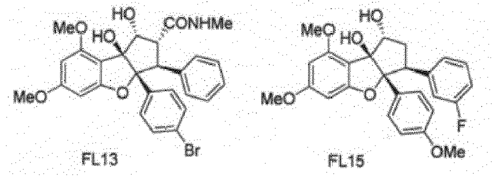

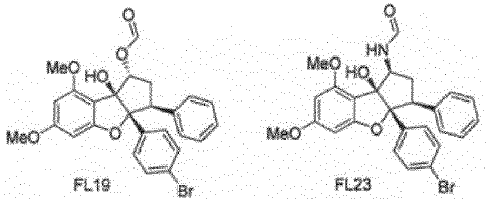

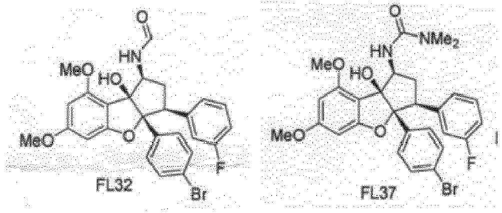

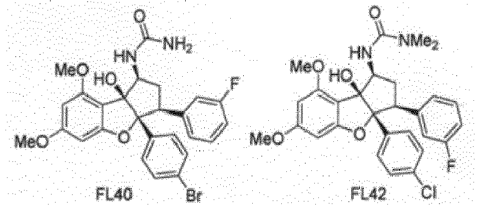

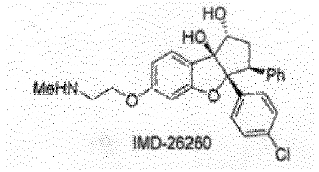

8. A pharmaceutical composition according to claim 6 or claim 7 for use in the prevention or treatment of a disease in which RAS signalling is pathologically involved, in particular a KRAS-mutated proliferative disorder or genetic disorder.

9. The pharmaceutical composition according to claim 8, wherein the disease is a neoproliferative disease, cancer, RASOpathy or craniofacial syndrome.

10. The pharmaceutical composition according to claim 9, wherein the cancer is colorectal cancer, lung cancer, hematological cancer, MYH associated polyposis, bladder cancer, melanoma, acute myeloid leukemia (AML), or pancreatic cancer.

11. The pharmaceutical composition according to claim 8, wherein RAS signalling involves a mutated KRAS gene, in particular a G12V, G12C, G12D, G13C, G13D, G13S, Q61H, Q61R, Q61K mutation.

12. A flavagline derivative having the general formula (I) 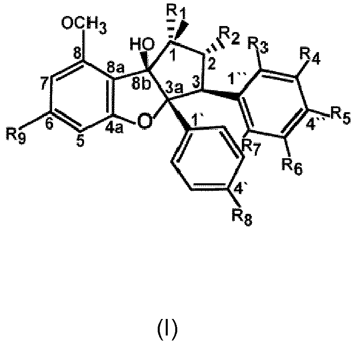 in which
R1 is HO, O-O, HNO, HNON(CH₃)₂, HNONH₂;
R2 is H, COOCH₃, CONHCH₃, NO(CH₃)₂;
R3, R4, R5 are each independently H, OH;
R6 is H, OH, F;
R7 is H, OH;
R8 is H, OCH₃, Br, F, Cl;
R9 is OCH₃, CH₃HN;
for use as a medicament.

13. The flavagline derivative according to claim 12, wherein the flavagline derivative is FL3, FL10, FL13, FL15, FL19, FL23, FL32, FL37, FL40, FL42, IMD-26260 having the following structures:
